# EUROPEAN PATENT APPLICATION

(11) **EP 4 837 964 A1**
(43) Date of publication of application: **11.11.2026**
(21) Application number: 25736264.0
(22) Date of filing: 02.01.2025
(51) Int. Cl.: C12N 15/113, C12N 15/86, A61K 31/7105, A61K 48/00, A61P 25/00, C07K 14/47

(54) **MECP2 TRANSCRIPT-TARGETING TRANS-SPLICING RIBOZYME, AND USE THEREOF**

(30) Priority: 02.01.2024 KR 20240000539
(71) Applicant: Rznomics Inc., Yongin-si, Gyeonggi-do 16890 (KR)
(72) Inventor: LEE, Seong Wook, Seoul 02780 (KR); HAN, Seung Ryul, Yongin-si Gyeonggi-do 16919 (KR); KIM, Ji Hyun, Seoul 03472 (KR)
(74) Representative: De Clercq & Partners
(86) International application number: PCT/KR2025/000061
(87) International publication number: WO 2025/147110

(57) **Abstract**

The present invention relates to a MeCP2 gene-specific trans-splicing ribozyme, and a use thereof. The trans-splicing ribozyme according to the present invention replaces the RNA of a mutant MeCP2 gene, which causes Rett syndrome, with a wild type MeCP2 RNA, so as to reduce the expression of the mutant MeCP2 protein and increase the expression of the wild type MeCP2 protein, and enables regulated expression so that wild type MeCP2 expression can be prevented from exceeding physiological levels, and thus can be effectively used for preventing or treating Rett syndrome.

## Description

### TECHNICAL FIELD

The disclosure relates to an MeCP2 gene-specific trans-splicing ribozyme and a use thereof.

### BACKGROUND ART

It has been reported that a group I intron ribozyme from *Tetrahymena thermophilla* is capable of inducing, in vitro, a trans-splicing reaction in which the ribozyme targets a 5' exon present in trans and then attaches, to the 5' exon, an exon RNA attached to the 3' end of the ribozyme, thereby connecting the 5' exon to a separately present transcript. This suggests that, through the trans-splicing reaction of a group I ribozyme, transcripts of genes associated with various genetic diseases may also be corrected, and indeed it has recently been revealed that, in erythrocyte precursor cells of a patient with sickle cell disease, a β-globin gene transcript may be converted by a trans-splicing ribozyme into an anti-sickling γ-globin gene transcript.

As a gene therapy method, a trans-splicing ribozyme may correct a mutant gene RNA into a normal RNA, and by the same principle, may reduce the expression of a given gene while simultaneously expressing a target gene in a host cell. In this case, because the ribozyme operates at the RNA level, the ribozyme has the advantage of not causing problems such as undesirable immune reactions and safety problems due to the incorporation of a gene at a random location within the host cell genome.

Meanwhile, Rett syndrome (RTT) is a neurodevelopmental disorder, and its incidence is relatively high, occurring in approximately one in 10,000 to 15,000 individuals. Rett syndrome is an X-linked dominantly inherited disease in which, after relatively normal development from 6 months to 18 months after birth, a decrease in head growth occurs along with loss of acquired cognitive and motor abilities, loss of language function, and characteristic hand stereotypies in which hand-washing-like movements are repeated. A family history is rare, the disease occurs only in girls, and boys having the abnormality are known to die at birth.

It is known that, in 96% to 98% of patients diagnosed with Rett syndrome, the cause is a mutation in the MeCP2 gene, which is located on the X chromosome (Xq28) and encodes Methyl-CpG binding protein 2 (MeCP2). The MeCP2 protein is important for the development of the nervous system, particularly the brain. A mutation in the MeCP2 gene reduces production of the MeCP2 protein or causes incomplete MeCP2 protein production, and as a result, prevents normal development of a given region of the brain responsible for the functions of sensation, emotion, and the motor and autonomic nervous systems. According to what is known, there are eight major MeCP2 mutations in Rett syndrome, and the location and type of the mutation affect the onset and severity of the syndrome symptoms, among which the MeCP2 T158M mutation and the MeCP2 R106W mutation are the most common. In addition, MeCP2 duplication syndrome is caused by MeCP2 overexpression due to duplication of the MeCP2 gene, and presents with abnormalities in cognitive function and neural development as its symptoms. MeCP2 duplication syndrome occurs in boys and is mainly inherited maternally, but it is also known to occur sporadically in rare cases.

That is, while the expression of WT MeCP2, which is able to perform its intrinsic functions, is also important for the treatment of Rett syndrome, the excessive expression of WT MeCP2 must be controlled. Accordingly, the present inventors conducted intensive research into a method capable of treating Rett syndrome without inducing excessive WT MeCP2 expression in cells using a trans-splicing ribozyme targeting MeCP2, and thereby completed the disclosure.

### SUMMARY OF THE INVENTION

### TECHNICAL GOALS

The technical problem to be achieved by the disclosure is to provide an MeCP2 transcript-specific trans-splicing ribozyme that has excellent safety and excellent expression efficiency of a normal MeCP2 RNA and that is able to regulate the amount of expression, as well as a use thereof for treating Rett syndrome.

However, technical goals to be achieved by the disclosure are not limited to those described above, and other goals not mentioned above may be clearly understood by one of ordinary skill in the art from the following description.

### TECHNICAL SOLUTIONS

In order to solve the above problem, the disclosure provides a trans-splicing ribozyme that targets an MeCP2 RNA.

In an embodiment of the disclosure, the trans-splicing ribozyme may have a structure of 5'- IGS (internal guide sequence)-Ribozyme*-3'.

In another embodiment of the disclosure, the trans-splicing ribozyme may have a structure of 5'-IGS (internal guide sequence)-Ribozyme*-exon-3'.

In another embodiment of the disclosure, the IGS region may consist of a nucleotide sequence with a length of 2 nt to 10 nt capable of specifically binding to MeCP2 RNA and forming a G/U wobble base pair, and preferably, the IGS region may consist of a nucleotide sequence with a length of 2 nt to 10 nt capable of complementarily binding to a region including position +18, +31, +33, +36, +45, +54, +127, +145, +148, +162, +167, +181, +188, +211, +294, +289, +296, +304, +314, or +357 of MeCP2 RNA and forming a G/U wobble base pair.

In another embodiment of the disclosure, the IGS region may include or consist of the nucleotide sequence of SEQ ID NO: 3, 5, 7, 9, 11, 13, 15, 17, 19, 21, 23, 25, 27, 29, 31, 33, 35, 37, 39, or 61.

Specifically, when the reaction site of the ribozyme is +18 of MeCP2 RNA, the IGS region may include or consist of the nucleotide sequence of SEQ ID NO: 3, when the reaction site is +31, the IGS region may include or consist of the nucleotide sequence of SEQ ID NO: 61, when the reaction site is +33, the IGS region may include or consist of the nucleotide sequence of SEQ ID NO: 5, when the reaction site is +36, the IGS region may include or consist of the nucleotide sequence of SEQ ID NO: 7, when the reaction site is +45, the IGS region may include or consist of the nucleotide sequence of SEQ ID NO: 9, when the reaction site is +54, the IGS region may include or consist of the nucleotide sequence of SEQ ID NO: 11, when the reaction site is +127, the IGS region may include or consist of the nucleotide sequence of SEQ ID NO: 13, when the reaction site is +145, the IGS region may include or consist of the nucleotide sequence of SEQ ID NO: 15, when the reaction site is +148, the IGS region may include or consist of the nucleotide sequence of SEQ ID NO: 17, when the reaction site is +162, the IGS region may include or consist of the nucleotide sequence of SEQ ID NO: 19, when the reaction site is +167, the IGS region may include or consist of the nucleotide sequence of SEQ ID NO: 21, when the reaction site is +181, the IGS region may include or consist of the nucleotide sequence of SEQ ID NO: 23, when the reaction site is +188, the IGS region may include or consist of the nucleotide sequence of SEQ ID NO: 25, when the reaction site is +211, the IGS region may include or consist of the nucleotide sequence of SEQ ID NO: 27, when the reaction site is +289, the IGS region may include or consist of the nucleotide sequence of SEQ ID NO: 29, when the reaction site is +294, the IGS region may include or consist of the nucleotide sequence of SEQ ID NO: 31, when the reaction site is +296, the IGS region may include or consist of the nucleotide sequence of SEQ ID NO: 33, when the reaction site is +304, the IGS region may include or consist of the nucleotide sequence of SEQ ID NO: 35, when the reaction site is +314, the IGS region may include or consist of the nucleotide sequence of SEQ ID NO: 37, and when the reaction site is +357, the IGS region may include or consist of the nucleotide sequence of SEQ ID NO: 39.

In another embodiment of the disclosure, the trans-splicing ribozyme may further include an antisense sequence (AS) region capable of complementarily binding to a portion of MeCP2 RNA, upstream of the IGS region.

In another embodiment of the disclosure, the length of the AS region may be 10 nt to 300 nt.

In another embodiment of the disclosure, the trans-splicing ribozyme may further include a nucleotide sequence extended in a 5' direction of the IGS region, wherein a portion of the extended nucleotide sequence may consist of a nucleotide sequence capable of binding complementarily to an MeCP2 RNA together with the IGS region to form a P1 helix, and a portion of the extended nucleotide sequence may consist of a nucleotide sequence capable of binding complementarily to a nucleotide sequence in a 5' direction of the exon region to form a P10 helix.

The disclosure confirmed the high trans-splicing efficiency by the trans-splicing ribozymes targeting +33, +45, or +304, selected the trans-splicing ribozyme targeting +45 as a random representative, designed various P10 structures in the trans-splicing ribozyme targeting +45, and confirmed the superiority of ribozymes v1 and v2 in SH-SY5Y cells, which are a neuroblastoma cell line.

Therefore, in the ribozyme of the disclosure, the portion of the extended nucleotide sequence that forms P10 may include or consist of the nucleotide sequence of SEQ ID NO: 43, 45, 47, or 49.

In addition, the disclosure provides a trans-splicing ribozyme expression vector including a nucleotide sequence of the trans-splicing ribozyme and/or a nucleotide sequence complementary thereto.

In an embodiment of the disclosure, the expression vector may further include a promoter operatively linked to the ribozyme gene, the promoter may be a neuron-specific promoter, and the nerve cells include a neuron, an astrocyte, and an oligodendrocyte. The promoter is not limited thereto, and may be a viral promoter, a constitutively active promoter, a CAG promoter, or the like.

In addition, the disclosure provides a system for delivering the trans-splicing ribozyme, or a gene delivery system including a trans-splicing ribozyme expression vector.

In addition, the disclosure provides a pharmaceutical composition for preventing or treating Rett syndrome, the pharmaceutical composition including, as an active ingredient, at least one selected from the group consisting of the trans-splicing ribozyme, a ribozyme delivery system, a vector capable of expressing a ribozyme, and a gene delivery system including the vector.

In addition, the disclosure provides a method of preventing or treating Rett syndrome, the method including administering, to a subject, at least one selected from the group consisting of the trans-splicing ribozyme, the trans-splicing ribozyme expression cassette, a vector capable of expressing the trans-splicing ribozyme, and a gene delivery system including the trans-splicing ribozyme, the expression cassette, or the vector.

In an embodiment of the disclosure, the gene delivery system may be a viral or non-viral gene delivery system.

In addition, the disclosure provides a use of the trans-splicing ribozyme, a ribozyme expression cassette, a vector capable of expressing a ribozyme, or the gene delivery system, for manufacturing a medicament for preventing or treating Rett syndrome.

### EFFECTS OF THE INVENTION

The trans-splicing ribozyme, according to the disclosure, may be usefully used for preventing or treating Rett syndrome, the trans-splicing ribozyme being capable of replacing the RNA of a mutant MeCP2 gene that causes the onset of Rett syndrome with wild-type MeCP2 RNA, thereby lowering the expression of a mutant MeCP2 protein, increasing the expression of a wild-type MeCP2 protein, and regulating the expression of WT MeCP2 such that the WT MeCP2 expression does not exceed a physiological level.

### BRIEF DESCRIPTION OF DRAWINGS

FIG. 1 is a schematic diagram of the mechanism of action of a trans-splicing ribozyme that targets MeCP2 RNA.
FIG. 2 shows a structure of a vector for expression of a trans-splicing ribozyme targeting MeCP2 RNA.
FIG. 3 is a schematic diagram illustrating the experimental procedure for selecting a target site of MeCP2 RNA through mapping.

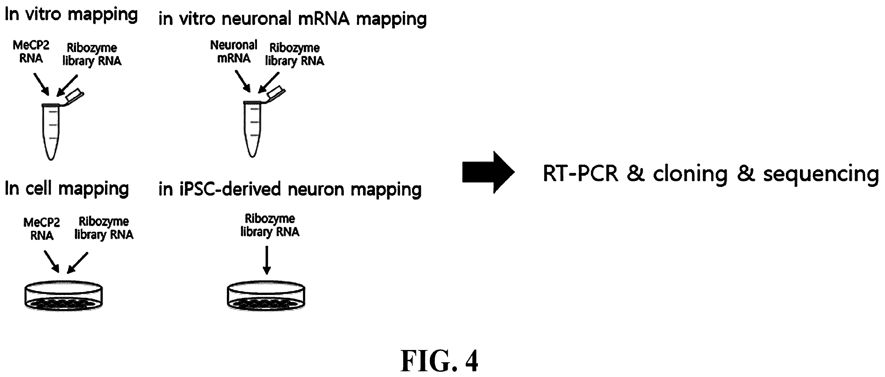

FIG. 4 is a schematic diagram of an experiment for reaction site mapping of MeCP2 RNA in vitro and in cells.
FIG. 5a shows results of reaction site mapping in vitro and in cells.
FIG. 5b shows IGS of a reaction site target selected from the mapping results and the target site sequence thereof.
FIG. 6 is a schematic diagram of various variants of MeCP2 RNA transcripts. The red block is a 5' UTR region, the blue block is an ORF, and the blue arrow indicates the position of the start codon (ATG).
FIG. 7 shows results of performing an in vitro trans-splicing reaction by preparing a +33, +45, or +304 target ribozyme.
FIG. 8 shows ribozyme designs for a +45 target site having various P10 structures.
FIGS. 9a and 9b show results of confirming a trans-splicing reaction of MeCP2 mRNA by a +45 target ribozyme having various P10 structures in SH-SY5Y cells, where FIG. 9b shows the results of confirming whether a trans-splicing product was generated, and FIG. 9c shows results of confirming a reaction site at which trans-splicing occurred in the trans-splicing product.
FIG. 10 shows results of confirming ribozyme action on the +45 target site having various P10 structures in 293A cells.
FIGS. 11a to 11d show results of co-transfecting an MeCP2 E1 expression vector and a +45 target ribozyme expression vector having various P10 structures in SH-SY5Y cells, confirming the levels of MeCP2 E1, MeCP2 E2, total MeCP2, ribozyme, and trans-splicing product (TSP, the substituted RNA), and confirming the trans-splicing reaction site by the ribozyme.
FIGS. 12a to 12d show results of co-transfecting an MeCP2 E2 expression vector and a +45 target ribozyme expression vector having various P10 structures in SH-SY5Y cells, confirming the levels of MeCP2 E1, MeCP2 E2, total MeCP2, ribozyme, and TSP, and confirming the trans-splicing reaction site by the ribozyme.
FIG. 13 shows results of confirming the action of a +45 target ribozyme having various P10 structures in an iPSC-derived neuron.

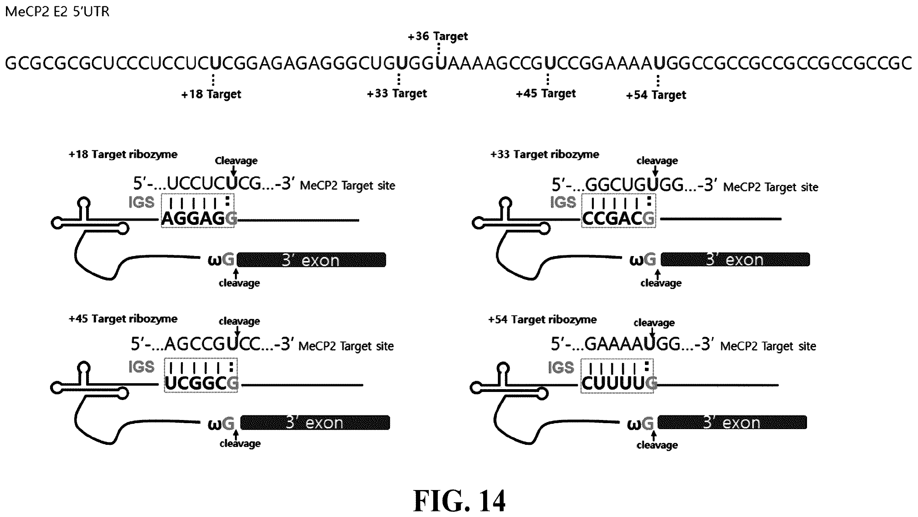

FIG. 14 is a schematic diagram of an example of the trans-splicing reaction region of a +18, +33, +45, or +54 target ribozyme and a target MeCP2 transcript.

### BEST MODE(S) FOR CARRYING OUT THE INVENTION

From the statistical finding that MeCP2 mutations are frequent among Rett syndrome patients, the present inventors intensively studied a gene therapy method for the prevention and treatment of Rett syndrome targeting MeCP2 mutations, thereby completing the disclosure.

Specifically, in order to secure a target region capable of effectively inducing trans-splicing in a ribozyme targeting MeCP2 RNA, the present inventors mixed MeCP2 RNA with a random library ribozyme and, through sequence analysis of the resulting trans-splicing products, identified the +18, +31, +33, +36, +45, +54, +127, +145, +148, +162, +167, +181, +188, +211, +294, +289, +296, +304, +314, and +357 bases as reaction sites.

In addition, MeCP2 has various variants. Therefore, the present inventors selected a target site capable of covering all variants, in addition to the results obtained through in vitro mapping and the results confirming the trans-splicing effect of the selected ribozyme.

The present inventors produced ribozymes specific for the +33, +45, or +304 targets among the target sites identified through the mapping process and performed an in vitro trans-splicing reaction, and as a result, it was confirmed that the ribozyme of each reaction site target underwent a trans-splicing reaction with MeCP2 RNA to produce a reaction product.

Furthermore, each ribozyme expression vector was transfected into SH-SY5Y cells, which are MeCP2-positive cells, and the levels of E1 MeCP2, E2 MeCP2, and E1/E2 total MeCP2 were measured, and as a result, the expression vectors transfected into the cells expressed the ribozymes, and the expressed ribozymes were confirmed to react with MeCP2 RNA accurately at the target sites. In the present specification, the results for the +45 target ribozyme are presented as a representative example.

In addition, the present inventors designed various P10 structures to improve the efficiency of the trans-splicing reaction with the target, and confirmed the reaction by transfecting MeCP2-positive cells with ribozyme expression vectors having the various P10 structures. As a result, the P10 structures exhibited differences in efficiency depending on whether the experiment involved endo MeCP2 or exo MeCP2, or depending on the cell line. Specifically, when endo MeCP2 was targeted in 293A cells, high levels of trans-splicing product were observed with ribozyme v2 and ribozyme v4, and when exo MeCP2 was targeted in SH-SY5Y cells, high levels of trans-splicing product were observed with ribozyme v1 and ribozyme v2. Taken together, it was confirmed that v1, v2, and v4 exhibited excellent trans-splicing efficiency.

The present inventors provide a trans-splicing ribozyme for reducing the expression of mutant MeCP2 protein for treating Rett syndrome, wherein the ribozyme may have a structure of 5'-IGS (internal guide sequence)-Ribozyme*-3', and may have a structure of 5'-IGS (internal guide sequence)-Ribozyme*-exon-3' in which a target gene is linked in the 3' direction. In addition, the ribozyme may further include an antisense sequence (AS) region having a sequence complementary to MeCP2 RNA in the 5' direction of the IGS region in order to increase the trans-splicing efficiency.

The ribozyme of the disclosure may include extended nucleotides at the front (5' direction) of the IGS to form a P1 helix region and/or a P10 helix region. The P1 helix is a secondary structure formed by complementary binding between the target MeCP2 RNA and both the IGS region of the group I intron ribozyme and three nucleotides extending upstream thereof, and is capable of increasing the binding specificity and binding affinity of the ribozyme to a target. The P10 helix is a secondary structure formed by complementary binding between a nucleotide sequence of 5 nt to 10 nt in length extending in the upstream direction of the IGS region of the ribozyme and a nucleotide sequence in the 5' direction of an exon region included in the ribozyme, and is capable of increasing the trans-splicing efficiency of the ribozyme.

In this specification, a nucleotide sequence that forms the P1 helix is referred to as a P1 region or a P1 helix region, and likewise, a nucleotide sequence that forms the P10 helix is referred to as a P10 region or a P10 helix region.

The term "ribozyme" used in the disclosure refers to an RNA molecule that acts like an enzyme or a molecule consisting of a protein containing such an RNA molecule, and is also called an RNA enzyme or a catalytic RNA. An RNA molecule having a definite tertiary structure performs chemical reactions and has catalytic or autocatalytic properties. Some ribozymes are known to cleave themselves or other RNA molecules to inhibit activity, and other ribozymes are known to catalyze the aminotransferase activity of ribosomes. Such ribozymes may include a hammerhead ribozyme, a VS ribozyme, and a hairpin ribozyme.

In this specification, "trans-splicing" means linking RNAs from different genes to each other.

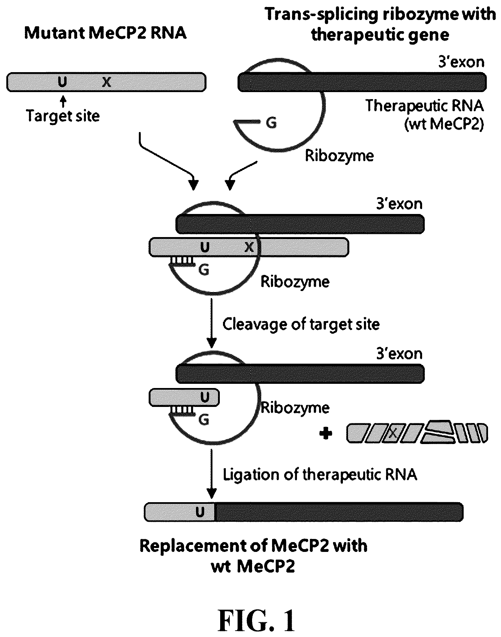

In this specification, a ribozyme refers to a trans-splicing ribozyme that cleaves MeCP2 RNA and replaces MeCP2 RNA with its own RNA (in particular, the 3' exon region of the ribozyme), thereby inhibiting the expression of a mutant MeCP2 protein in a host cell and inducing the expression of a gene encoded in the exon region (FIG. 1). In the disclosure, the ribozyme may have a structure of 5'-IGS (internal guide sequence)-Ribozyme^{*}-exon-3' or 5'-AS (antisense)-IGS-Ribozyme^{*}-exon-3'. The Ribozyme* region in the structure refers to a region that, within the trans-splicing ribozyme, performs the function of cleaving MeCP2 RNA and/or replacing MeCP2 RNA with an exon region. Unless otherwise specified herein, the ribozyme refers to a trans-splicing ribozyme that includes the entire structure described above, including the Ribozyme^{*} region, which is a region that performs substantial functions.

In the disclosure, the gene encoded in the 3' exon region of the ribozyme may be a wild-type MeCP2 gene, or may not encode a protein. Because the substitution action of the trans-splicing ribozyme is dependent on the target RNA level, the gene encoded in the exon region of the ribozyme is expressed in a manner dependent on the target RNA level.

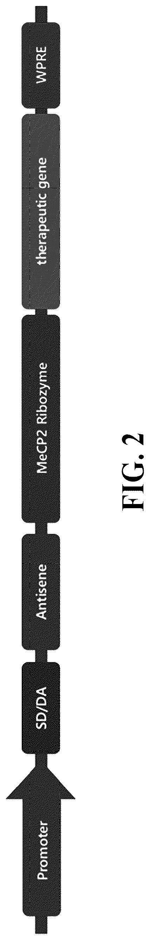

In addition, the disclosure provides a vector that expresses the trans-splicing ribozyme targeting the MeCP2 transcript. The vector includes a trans-splicing ribozyme expression cassette targeting the MeCP2 transcript, and the expression cassette may include a splicing donor/splicing acceptor sequence (SD/SA sequence) linked, in the 5' direction, to the trans-splicing ribozyme coding region in order to increase the expression efficiency of the ribozyme, and may include a woodchuck hepatitis virus posttranscriptional regulatory element (WPRE) linked, in the 3' direction, to the trans-splicing ribozyme coding region (FIG. 2).

As used herein, "vector" refers to a gene construct that is an expression vector capable of expressing a trans-splicing ribozyme in a suitable host cell, the gene construct including essential regulatory elements operably linked so that a ribozyme gene insert contained in the vector is expressed.

In the disclosure, "operably linked" refers to a functional linkage between a nucleic acid expression regulatory sequence that performs a general function and a nucleic acid sequence encoding a target gene.

For example, when a ribozyme coding sequence is operably linked to a promoter, expression of the ribozyme coding sequence is under the influence or regulation of the promoter. Two nucleic acid sequences (the ribozyme coding sequence and a promoter region sequence at the 5' end of this sequence) are operably linked when promoter action is induced and the ribozyme coding sequence is transcribed, and the two sequences are considered to be operably linked when the linkage characteristics between the two sequences do not induce a frameshift mutation and the expression regulatory sequence does not inhibit the expression of the ribozyme. An operable linkage with a recombinant vector may be prepared using gene recombination techniques well known in the art, and site-specific DNA cleavage and ligation may utilize enzymes and the like generally known in the art.

The vector, according to the disclosure, includes, in addition to expression regulatory elements such as a promoter, an operator, an initiation codon, a termination codon, a polyadenylation signal, and an enhancer, a signal sequence or a leader sequence for membrane targeting or secretion, and may be prepared in various ways depending on the purpose. The promoter of the vector may be constitutive or inducible. In addition, the expression vector includes a selectable marker for selecting a host cell containing the vector, and may include an origin of replication in the case of a replicable expression vector. The vector may be self-replicating or may be integrated into host DNA.

The vector, according to the disclosure, preferably may be a plasmid vector, a cosmid vector, or a viral vector, and most preferably may be a viral vector. The viral vector preferably may be a vector derived from a retrovirus, for example, human immunodeficiency virus (HIV), murine leukemia virus (MLV), avian sarcoma/leucosis virus (ASLV), spleen necrosis virus (SNV), Rous sarcoma virus (RSV), or mouse mammary tumor virus (MMTV), an adenovirus, an adeno-associated virus (AAV), or a herpes simplex virus (HSV), but is not limited thereto. The recombinant vector, according to the disclosure, may most preferably be a recombinant adeno-associated virus vector.

The term "promoter" used in the disclosure is a part of DNA and is involved in the binding of an RNA polymerase so as to initiate transcription. The promoter is generally located adjacent to and upstream of a target gene, and is a site to which an RNA polymerase or a transcription factor, which is a protein that induces the RNA polymerase, binds, and is capable of guiding the enzyme or protein to be positioned at the correct transcription initiation site. That is, the promoter is located at the 5' region of a gene to be transcribed on a sense strand and has a specific gene sequence that guides the RNA polymerase to bind to the corresponding position directly or through a transcription factor to initiate mRNA synthesis for the target gene.

The promoter, according to the disclosure, may be a viral promoter, a constitutively active promoter, a CAG promoter, or, from the standpoint of enhancing the expression of a gene in nerve cells, a nerve cell type-specific promoter, and the nerve cells include neurons, astrocytes, and oligodendrocytes.

As used herein, "gene delivery system" refers to a system capable of increasing the expression efficiency by enhancing the delivery efficiency of ribozyme RNA or a ribozyme gene and/or a nucleic acid sequence into the interior of a cell, and may be classified into a virus-mediated system and a non-viral system.

Viral-mediated systems use viral vectors such as a retroviral vector, an adenoviral vector, an adeno-associated viral vector, and a herpes simplex virus, and because such viral-mediated systems utilize the cell-penetrating mechanism inherent to the virus that causes infection in human cells, they are known to have a relatively higher intracellular gene delivery efficiency than non-viral systems. In addition, after entering a cell, non-viral vectors have a problem in that genes are degraded in endolysosomes after endosomes fuse with lysosomes; however, viral vectors have an advantage of high gene delivery efficiency due to low gene loss by a mechanism of delivering genes into the nucleus without passing through lysosomes.

The viral vectors that may be used in the disclosure function as gene delivery vehicles and, as described above for the recombinant vector, may be vectors derived from a retrovirus, an adenovirus, an adeno-associated virus, a herpes simplex virus, and the like. Such viral vectors may be introduced into the inside of a cell by a transduction method, such as infection, after being assembled into viral particles.

The non-viral system is a method using a cationic lipid carrier or a cationic polymer carrier as a delivery vehicle for a nucleic acid and/or a gene, or using an LNP or electroporation.

A cationic lipid carrier is a method in which the positive charge of a nanometer-sized liposome or lipid-based nanoparticle composed mainly of a cationic lipid is used to form a complex with negatively charged ribozyme RNA, a gene, an expression vector containing a gene, or a nucleic acid, and then the complex is delivered into the cell by phagocytosis. The complex delivered into a cell is first delivered from the endosome to the lysosome, then escapes into the cytoplasm and is expressed. A cationic polymer carrier delivers ribozyme RNA or a gene in a manner similar to a cationic lipid carrier, except that a polymer is used instead of a lipid, and representative cationic polymers include polyethyleneimine, poly-L-lysine, and chitosan.

Accordingly, a complex formed by combining the recombinant vector of the disclosure with a cationic lipid carrier or a cationic polymer carrier may be used as a gene delivery vehicle.

In the disclosure, the gene delivery system includes the recombinant vector described above, and although both a viral-mediated system and a non-viral system may be used, it is preferable to use a viral-mediated system.

In addition, the trans-splicing ribozyme of the disclosure is capable of targeting MeCP2 RNA in a nerve cell to inhibit the expression of a mutant MeCP2 protein, and since an MeCP2 mutation is known as a major causative gene for the onset risk of Rett syndrome, the trans-splicing ribozyme of the disclosure, the ribozyme delivery system, the ribozyme expression vector, and the gene delivery system including the vector may be used in a pharmaceutical composition for preventing or treating Rett syndrome.

The term "prevention" as used in the disclosure refers to any action that suppresses or delays the onset of Rett syndrome through administration of the pharmaceutical composition according to the disclosure.

The term "treatment" as used herein means any action of improving Rett syndrome or beneficially altering the symptoms of Rett syndrome by administration of the pharmaceutical composition according to the disclosure.

The pharmaceutical composition according to the disclosure may further include pharmaceutically acceptable carriers, excipients, or diluents. Examples of pharmaceutically acceptable carriers, excipients, and diluents that can be used in the pharmaceutical composition of the disclosure may include lactose, dextrose, sucrose, sorbitol, mannitol, xylitol, erythritol, maltitol, starch, acacia gum, alginate, gelatin, calcium phosphate, calcium silicate, calcium carbonate, cellulose, methyl cellulose, polyvinylpyrrolidone, water, methylhydroxybenzoate, propylhydroxybenzoate, talc, magnesium stearate, mineral oil, and the like.

The pharmaceutical composition of the disclosure may be administered orally or parenterally according to the desired method, but parenteral administration is preferable.

According to an embodiment of the disclosure, the pharmaceutical composition, according to the disclosure, may be administered intravenously, intra-arterially, into brain tissue, subcutaneously, or by intracisternal injection or intrathecal injection. The injection, according to the disclosure, may be in a form dispersed in a sterile medium so that the injection can be used as is when administered to a patient, or may be in a form administered after adding distilled water for injection to disperse the injection to an appropriate concentration upon administration. Furthermore, when prepared as an injection, the injection may be mixed with buffering agents, preservatives, analgesics, solubilizers, isotonic agents, stabilizers, and the like, and may be prepared in unit-dose ampoule or multiple-dose forms.

The dosage of the pharmaceutical composition of the disclosure varies depending on the patient's condition and weight, degree of disease, drug form, route of administration, and time, but can be appropriately selected by those skilled in the art. In addition, the pharmaceutical composition, according to the disclosure, may be used alone, or may be used in combination with another therapeutic agent for Rett syndrome or another therapeutic method.

### MODE(S) FOR CARRYING OUT THE INVENTION

Hereinafter, embodiments will be described in detail with reference to the accompanying drawings. However, various modifications may be made to the embodiments, and the scope of the disclosure is not limited or restricted by the embodiments. It should be understood that all modifications, equivalents or substitutions to the embodiments are included within the scope of the disclosure.

The terminology used herein is for the purpose of describing particular embodiments only and is not to be limiting of the embodiments. The singular forms "a," "an," and "the" are intended to include the plural forms as well, unless the context clearly indicates otherwise. It will be further understood that the terms "comprises" and/or "comprising," when used in this specification, specify the presence of stated features, integers, steps, operations, elements, components or a combination thereof, but do not preclude the presence or addition of one or more other features, integers, steps, operations, elements, components, and/or groups thereof.

Unless otherwise defined herein, all terms used herein including technical or scientific terms have the same meanings as those generally understood by one of ordinary skill in the art. It will be further understood that terms, such as those defined in commonly used dictionaries, should be interpreted as having a meaning that is consistent with their meaning in the context of the relevant art and will not be interpreted in an idealized or overly formal sense unless expressly so defined herein.

When describing the embodiments with reference to the accompanying drawings, like reference numerals refer to like components and a repeated description related thereto will be omitted. In the description of embodiments, detailed description of well-known related technology will be omitted when it is deemed that such description will cause ambiguous interpretation of the disclosure.

### [EXAMPLES]

### Example 1. In vitro mapping

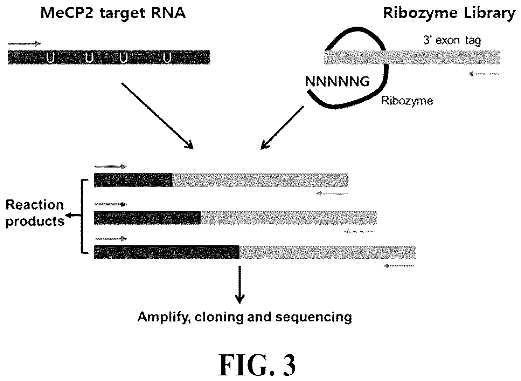

In order to secure various tools for removing MeCP2 mutations, MeCP2 RNA and a random library ribozyme were mixed and reacted in a test tube, RT-PCR was then performed, and the MeCP2 RNA site at which trans-splicing occurred was identified through sequence analysis of the PCR DNA band expected to be a trans-splicing product (FIG. 3).

Specifically, in vitro mapping and intracellular mapping were carried out using E1 MeCP2 RNA or E2 MeCP2 RNA and a ribozyme RNA library. For the in vitro mapping, MeCP2 RNA or intracellular mRNA and a random library ribozyme were mixed and reacted in a test tube, RT-PCR was then performed, and the MeCP2 RNA site at which trans-splicing occurred was identified through sequence analysis of the PCR DNA band expected to be a trans-splicing product. More specifically, MeCP2 RNA was prepared by synthesis through an in vitro transcription process, or was prepared by extracting mRNA from a cell expressing MeCP2, and a trans-splicing ribozyme RNA library having a random sequence (GNNNNN) at the 5'-terminal was synthesized through an in vitro transcription process. 0.1 pmole of MeCP2 RNA was mixed with a 1X in vitro trans-splicing reaction buffer (50 mM Hepes (pH 7.0), 150 mM NaCl, 5 mM MgCl2) to a total of 10 µL, and 1 pmole of a ribozyme library RNA was mixed with the 1X in vitro trans-splicing reaction buffer to a total of 9 µL, followed by adding 1 µL of 1 mM GTP to adjust the concentration to a final concentration of 0.1 mM GTP. These were each reacted at 95°C for 1 minute and at 37°C for 3 minutes, and after the two solutions were mixed, they were reacted at 37°C for 3 hours. The reaction solution was subjected to phenol extraction/EtOH precipitation to recover the RNA, and then RT-PCR was performed.

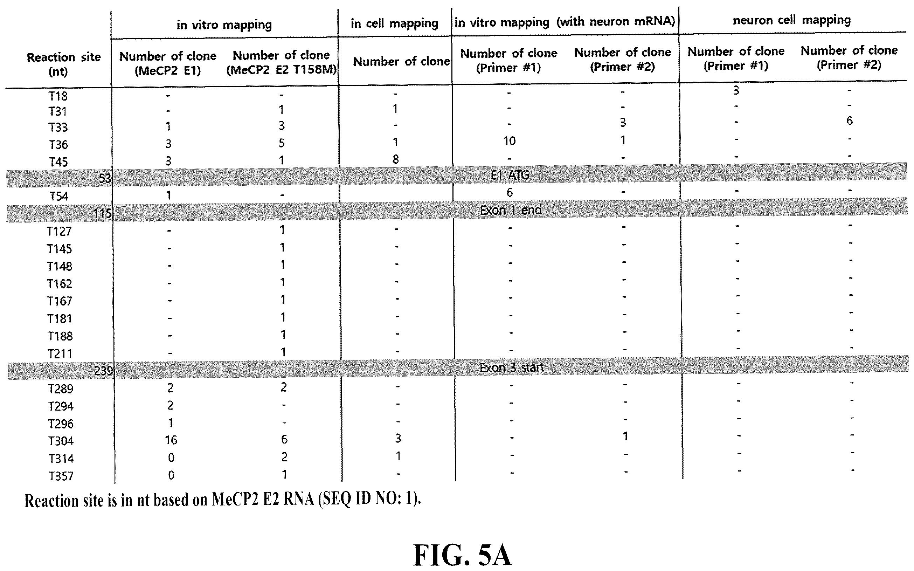

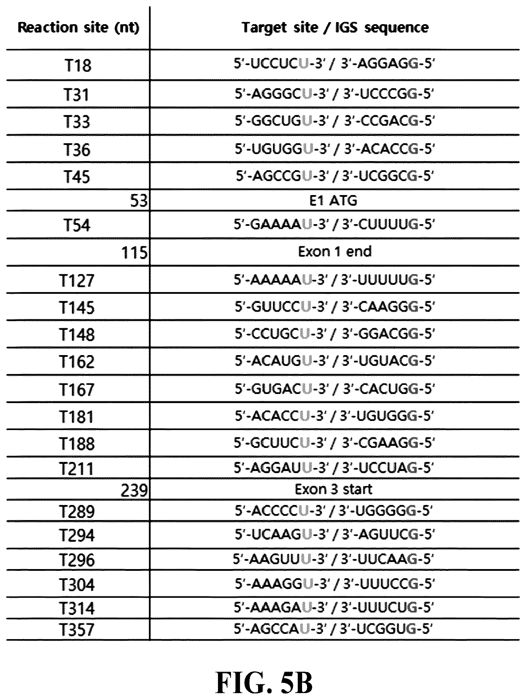

Subsequently, a ribozyme-specific RT primer (5'-ATGTGCTGCAAGGCGATT-3', SEQ ID NO: 56) was added, and then cDNA was synthesized by reverse transcription under a condition of a 20 uL volume. Using 2 uL of the synthesized cDNA, MeCP2 mRNA-specific 5' primer #1 (5'-GCGCGCGCTCCCTCCTCT-3', SEQ ID NO: 57) or 5' primer #2 (5'-GCGCGCGCTCCCTCCTCTCGGAGAG-3', SEQ ID NO: 58) and a ribozyme-specific 3' primer (5'-TGTAAAACGACGGCCAGTG-3', SEQ ID NO: 59) were each used at 10 pmole, 30-cycle PCR was performed under conditions of 98°C for 30 sec, 58°C for 30 sec, and 72°C for 30 sec, and the PCR band was identified on a 2% TAE agarose gel. Among the identified PCR bands, the bands showing differences compared to the MeCP2-only RNA sample were eluted through a gel extraction process, and the site of the MeCP2 RNA at which the trans-splicing reaction occurred was confirmed through nucleotide sequence analysis (FIGS. 5a and 5b). Considering, among the target sites identified through mapping, the targets that performed best in the mapping results or the utility in cells, the targeting efficiency for +33, +45, and +304 is considered to be the best.

### Example 2. Selection of the optimal target site among various MeCP2 variants

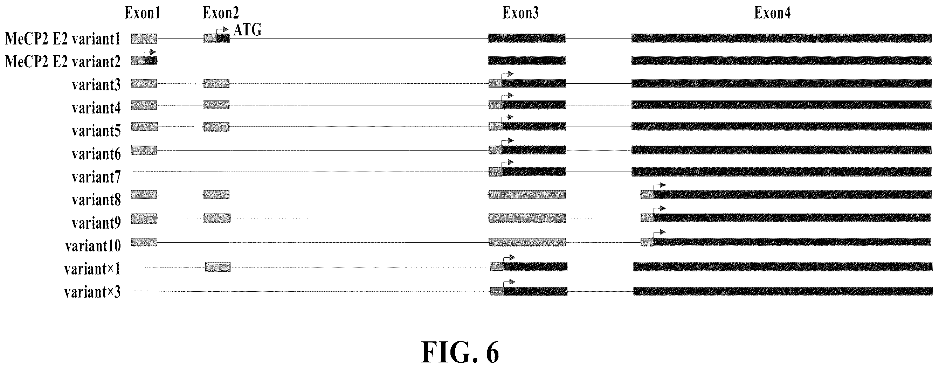

In addition, MeCP2 mRNA has various variants. Accordingly, the optimal target site is to be selected in order to produce a ribozyme capable of effectively performing trans-splicing action even on various MeCP2 mRNA variants. FIG. 6 is a schematic diagram indicating the untranslated region (UTR) and coding sequence (CDS) regions in a total of 12 types of MeCP mRNA variants.

The +18, +31, +33, +36, and +45 bases are located in Exon 1, and a ribozyme targeting the +18, +31, +33, +36, and +45 bases may target variants 1, 2, 3, 4, 5, 6, 8, 9, and 10.

The +127, +145, +148, +162, +167, +181, +188, and +211 bases are located in Exon 2, and a ribozyme targeting the +127, +145, +148, +162, +167, +181, and +211 bases may target variants 1, 3, 4, 5, 8, 9, and X1.

The +289, +294, +296, +304, +314, and +357 bases are located in Exon 3, and a ribozyme targeting the +294, +298, +304, +314, and +357 bases may target variants 1, 2, 3, 4, 5, 6, 7, 8, 9, 10, X1, and X3.

### Example 3. Confirmation of the trans-splicing effect of a ribozyme targeting each reaction site

Among the target sites identified through mapping, considering the target site that showed the best mapping result or the utility in cells, ribozymes targeting the +33, +45, and +304 sites, respectively, were prepared, and an in vitro trans-splicing reaction was performed to confirm the occurrence of trans-splicing.

More specifically, 0.1 pmole of MeCP2 E1 or E2 RNA was mixed with 1 pmole of each trans-splicing ribozyme library RNA to induce a trans-splicing reaction. RNA was recovered from each reaction solution and RT-PCR was performed, and the prepared cDNA was PCR-amplified using MeCP2 RNA-specific 5' primer #1 (5'-GCGCGCGCTCCCTCCTCT-3', SEQ ID NO: 57) and a ribozyme-specific 3' primer (5'-TGTAAAACGACGGCCAGTG-3', SEQ ID NO: 59) (95°C for 30 sec, 58°C for 30 sec, 72°C for 30 sec, 35 cycles), and each amplification product was confirmed by electrophoresis on an agarose gel.

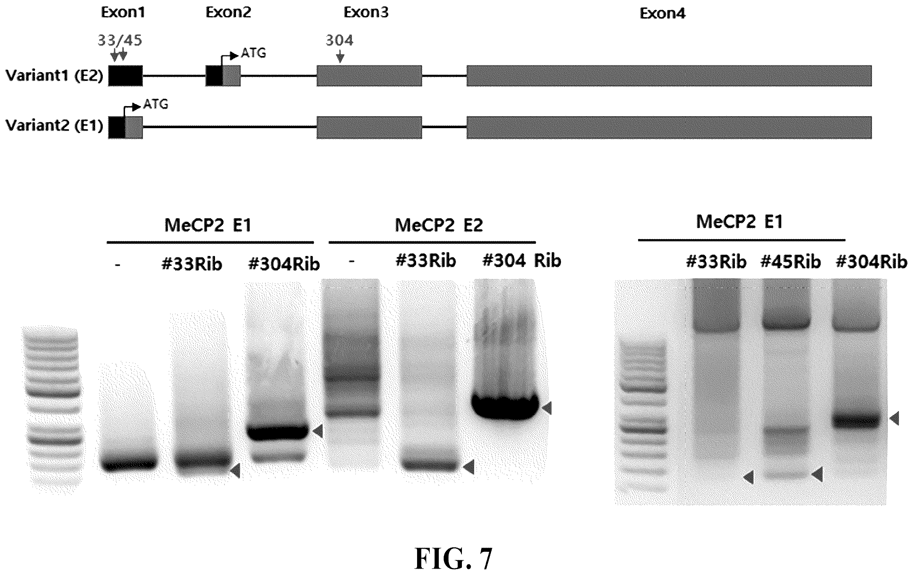

As a result, trans-splicing by the ribozymes targeting the +33, +45, and +304 sites of MeCP2 RNA could be confirmed (FIG. 7).

### Example 4. Design and preparation of a trans-splicing ribozyme targeting MeCP2 RNA +45

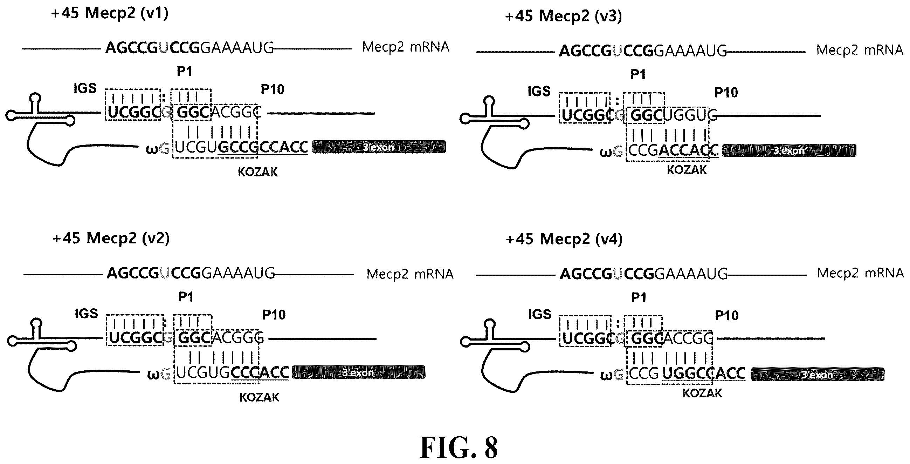

To confirm whether the ribozyme acts on the +45 target site among the target sites for which the in vitro trans-splicing reaction was confirmed, various forms of P10 were designed (FIG. 8).

### Example 5. Confirmation of the trans-splicing effect of MeCP2 RNA-targeting ribozymes

To confirm the effect of the +45-targeting ribozyme prepared in Example 4 above, confirmation was performed using SH-SY5Y cells, which are MeCP2-positive cells. An MeCP2-targeting ribozyme expression vector was transfected, and to confirm the trans-splicing effect, RNA was extracted and RT-PCR was performed.

Specifically, the cells were seeded at 4×10^5 cells in a 6-well plate and then cultured in a 37°C, 5% CO₂ incubator. 3 *µg* of a MeCP2-targeting ribozyme expression vector and 100 *µl* of Opti-MEM were placed into a 1.5 ml tube and mixed, and 9 *µl* of Fugene HD and 100 *µl* of Opti-MEM were placed into another 1.5 ml tube and mixed, after which the contents of the two tubes were mixed and kept at room temperature for 15 minutes to form a complex. After 15 minutes, the tube was centrifuged for 10 seconds, and then the contents were applied onto each of the cells for transfection, and after 4 hours the medium was replaced with fresh medium.

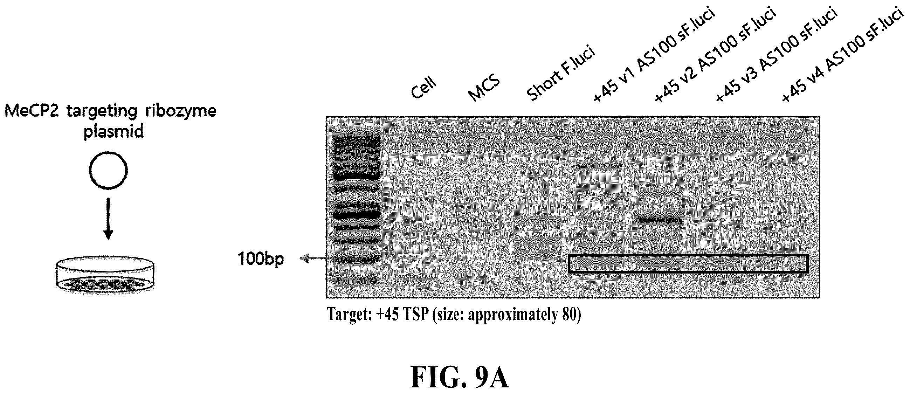

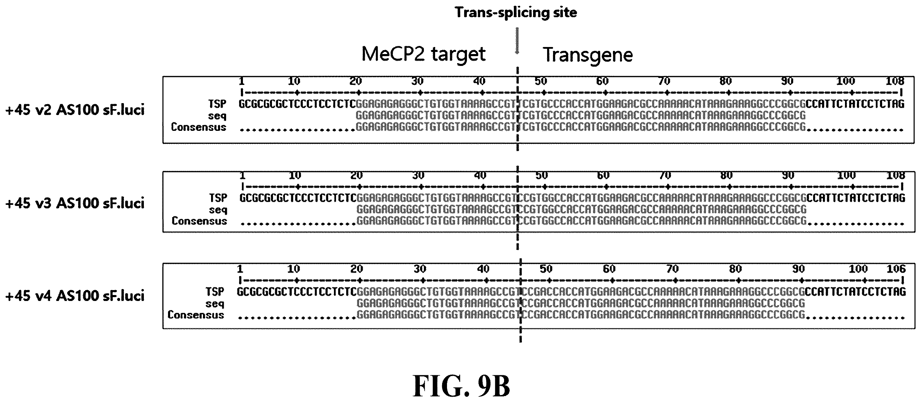

After culturing in an incubator at 37°C and 5% CO₂ for 48 hours, the cells were washed with 1X PBS, and treated with 500 µl of trizol to extract RNA. gDNA was removed by treating 5 *µg* of the extracted RNA with 1 *µl* of DNase I, and 1 *µg* thereof was reverse-transcribed to synthesize cDNA. Using the synthesized cDNA, the trans-splicing product was confirmed by PCR (FIG. 9a), and it was confirmed by nucleotide sequence analysis that the trans-splicing reaction had occurred accurately (FIG. 9b).

In addition, using 293A cells, which are another MeCP2-positive cell, the MeCP2-targeting ribozyme expression vector was transfected, and to confirm the trans-splicing effect, RNA was extracted, and RT-PCR was performed.

Specifically, the cells were seeded at 4×10^5 cells in a 6-well plate and then cultured in a 37°C, 5% CO₂ incubator. 3 *µg* of a MeCP2-targeting ribozyme expression vector and 100 *µl* of Opti-MEM were placed into a 1.5 ml tube and mixed, lipofectamine P3000 was dispensed at 6 *µl* each, and 4.5 *µl* of lipofectamine 3000 and 100 *µl* of Opti-MEM were placed into another 1.5 ml tube and mixed, after which the contents of the two tubes were mixed and kept at room temperature for 15 minutes to form a complex in the form of a liposome. After 15 minutes, the tube was centrifuged for 10 seconds, and then the contents were applied onto each of the cells for transfection, and after 4 hours the medium was replaced with fresh medium.

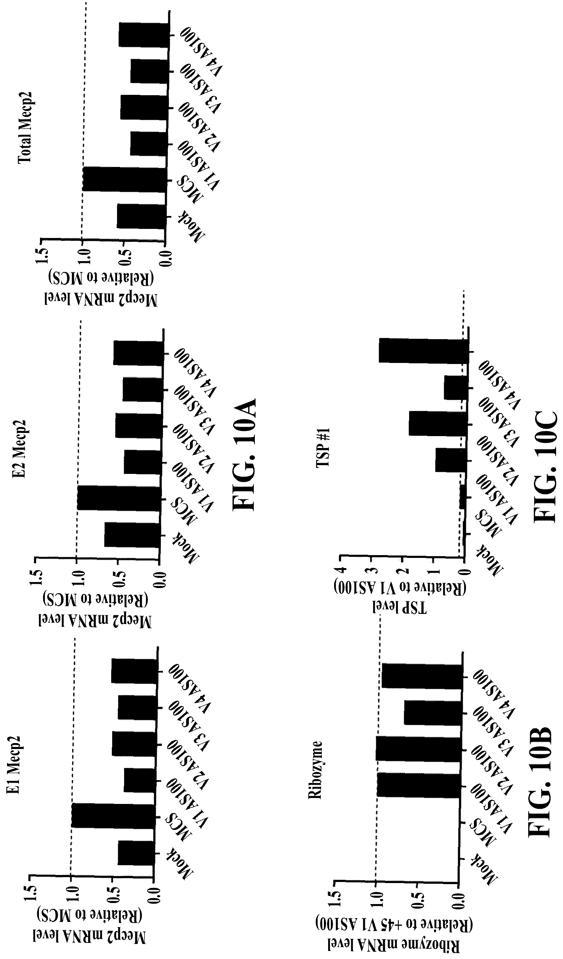

After culturing in an incubator at 37°C and 5% CO₂ for 48 hours, the cells were washed with 1X PBS, and treated with 500 µl of trizol to extract RNA. gDNA was removed by treating 5 *µg* of the extracted RNA with 1 *µl* of DNase I, and 1 *µg* thereof was reverse-transcribed to synthesize cDNA. Using the synthesized cDNA, the expression levels of E1 MeCP2, E2 MeCP2, and E1/E2 total MeCP2 were confirmed by qPCR, and it was confirmed that all the ribozymes had a reducing effect on endogenous E1 and E2 (FIG. 10a). Ribozyme expression did not show a large difference among the respective designs, and as a result of checking the trans-splicing products, although there were differences in the degree of production depending on the ribozyme design, trans-splicing products were confirmed to be produced for all designs, and among the compared constructs, the efficiency by v2 and v4 was judged to be relatively good (FIGS. 10b and 10c).

### Example 6. Confirmation of trans-splicing effect of MeCP2 RNA-targeting ribozyme on E1 and E2

To confirm the effect of the ribozyme on MeCP2 E1 and MeCP2 E2, the MeCP2-targeting ribozyme and the MeCP2 E1 or E2 expression vector were co-transfected into SH-SY5Y cells. Specifically, 3 *µg* of the MeCP2-targeting ribozyme expression vector and 0.5 *µg* of the MeCP2 expression vector were put into 100 *µl* of Opti-MEM in a 1.5 ml tube and mixed, 10.5 *µl* of Fugene HD and 100 *µl* of Opti-MEM were put into another 1.5 ml tube and mixed, and then the contents of the two tubes were mixed and left at room temperature for 15 minutes. Thereafter, RT-qPCR was performed after transfection by the same method as above.

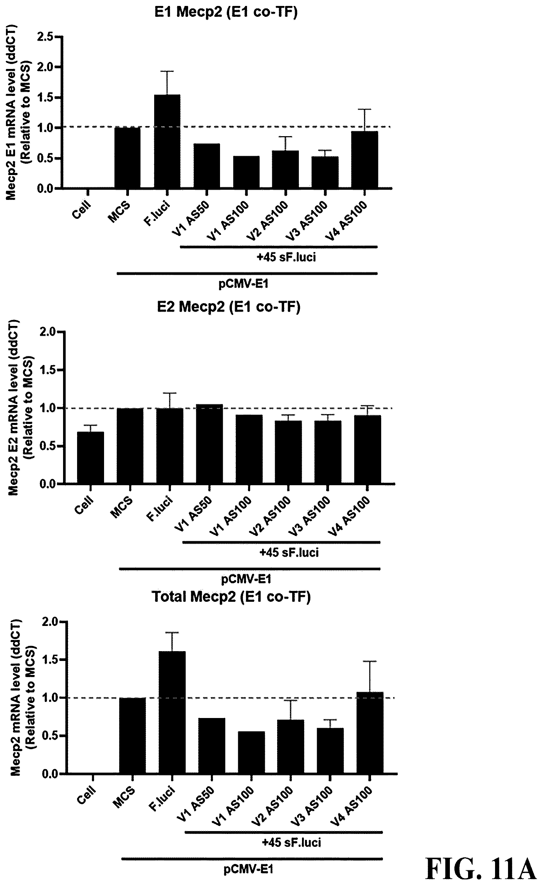

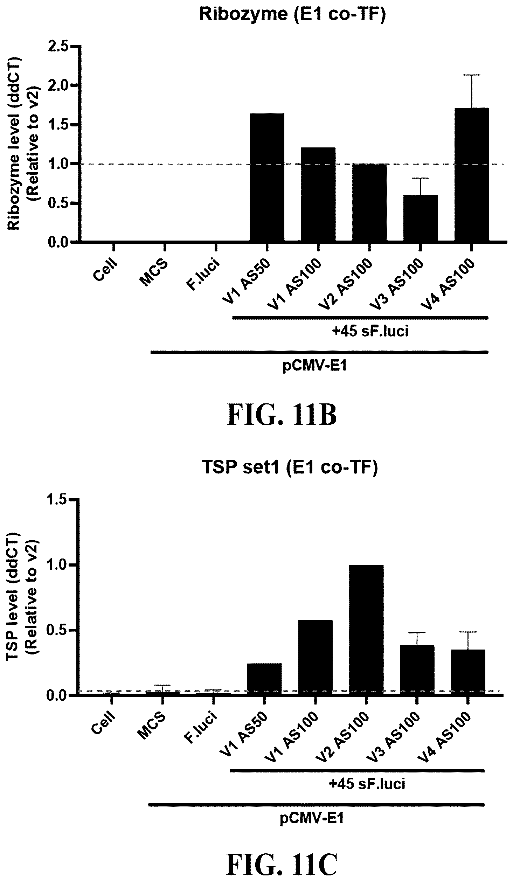

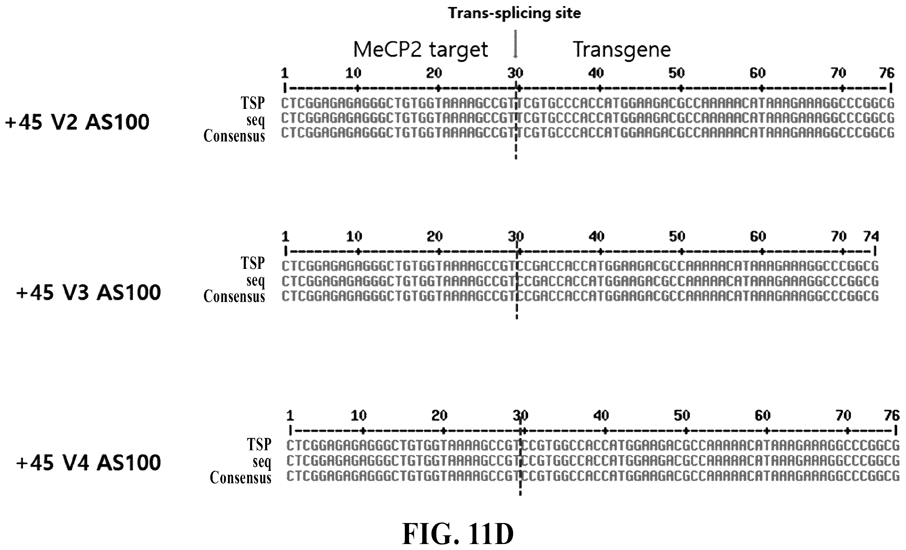

As a result, it was confirmed that when co-transfected with the E1 MeCP2 expression vector, the expressed E1 MeCP2 was reduced (FIG. 11a). In addition, although there were differences in ribozyme expression among the respective constructs, the ribozymes were normally expressed (FIG. 11b), and although there were differences in the amount of production, it was confirmed that trans-splicing products were produced (FIG. 11c). Sequence analysis was performed on the trans-splicing products confirmed by qPCR, and as a result, it was confirmed that trans-splicing occurred accurately at the targeted site, thereby confirming that the MeCP2 E1 was accurately replaced with the desired RNA (FIG. 11d).

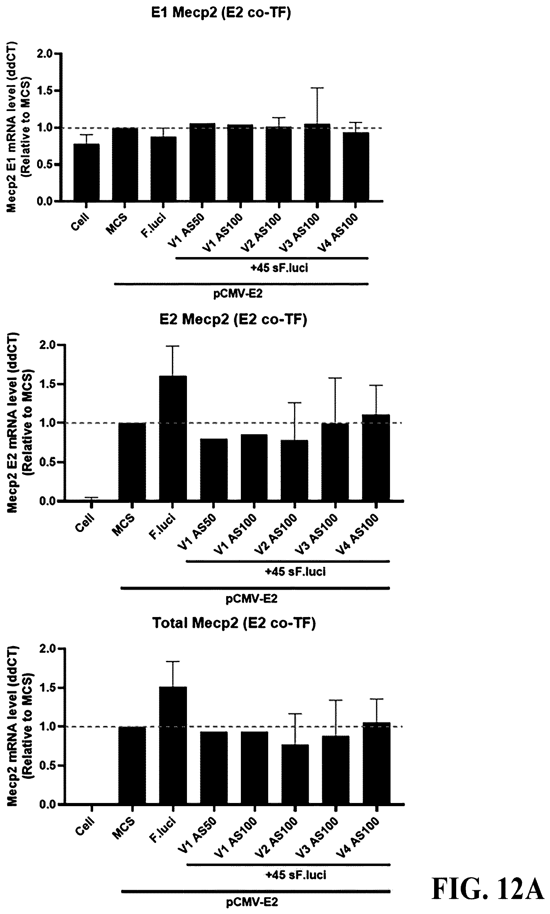

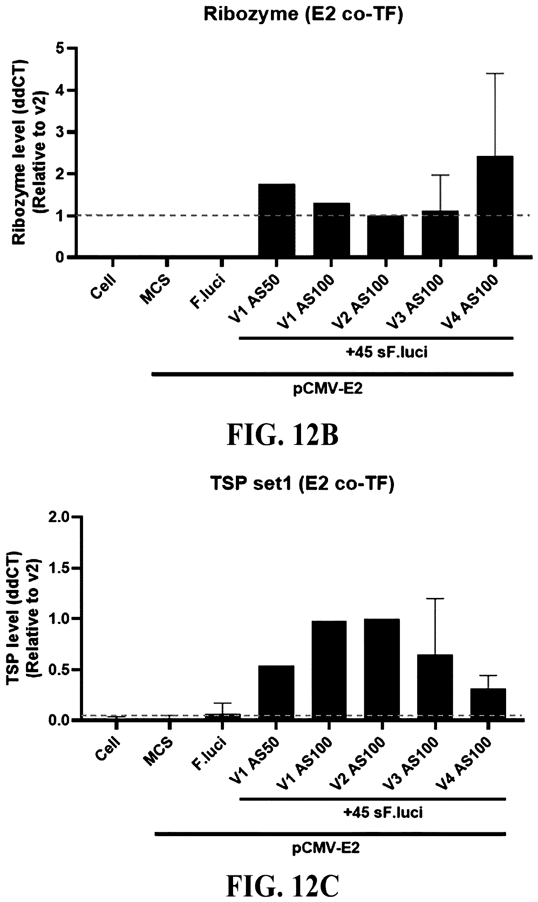

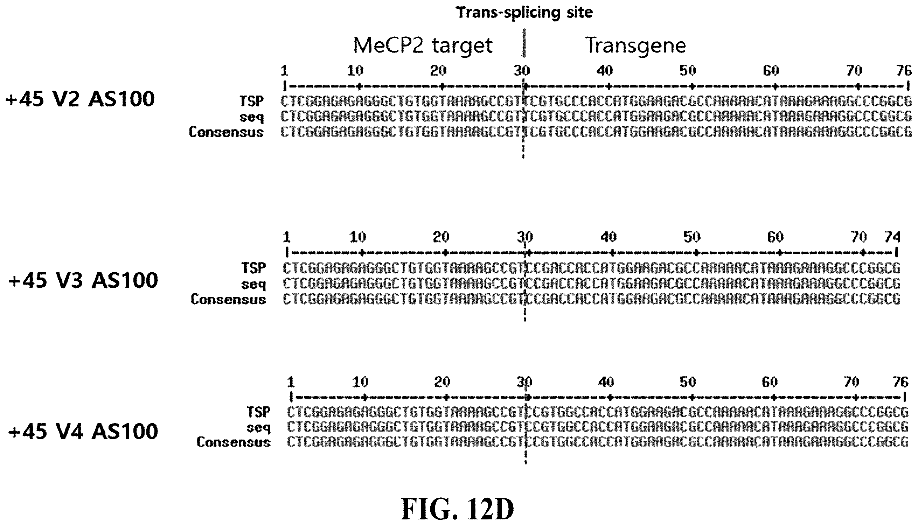

When co-transfection was performed with an E2 MeCP2 expression vector by the same method as the E1 MeCP2 regulation described above, the expressed E2 MeCP2 was confirmed to be reduced (FIGS. 12a to 12d).

Therefore, it can be seen that the trans-splicing ribozyme targeting MeCP2 RNA, according to the disclosure, does not correct only a given MeCP2 variant, but may replace the mutation site regardless of the variant.

### Example 7. Confirmation of the effect of an MeCP2 RNA-targeting ribozyme in neurons

Since Rett syndrome is a neurodevelopmental disorder and changes in proteins caused by mutations are known to affect neurons, iPSC-derived neuron cells were used to confirm the effect of the ribozyme in actual neurons. Two of the MeCP2-targeting ribozyme expression vectors were selected and transfected into neurons.

Specifically, 1.5x10^5 differentiated neurons were seeded in a 6-well plate and then cultured in a 37°C, 5% CO₂ incubator. After maturation for three weeks, the MeCP2-targeting ribozyme expression vector was transfected using Lipofectamine^{™} Stem Transfection Reagent. 1.25 *µg* of the MeCP2-targeting ribozyme expression vector and 100 *µl* of Opti-MEM were placed in a 1.5 ml tube and mixed, 5 *µl* of Lipofectamine^{™} Stem Transfection Reagent and 100 *µl* of Opti-MEM were placed in another 1.5 ml tube and mixed, and then the contents of the two tubes were combined and left at room temperature for 10 minutes. Thereafter, RT-qPCR was performed after transfection by the same method as above.

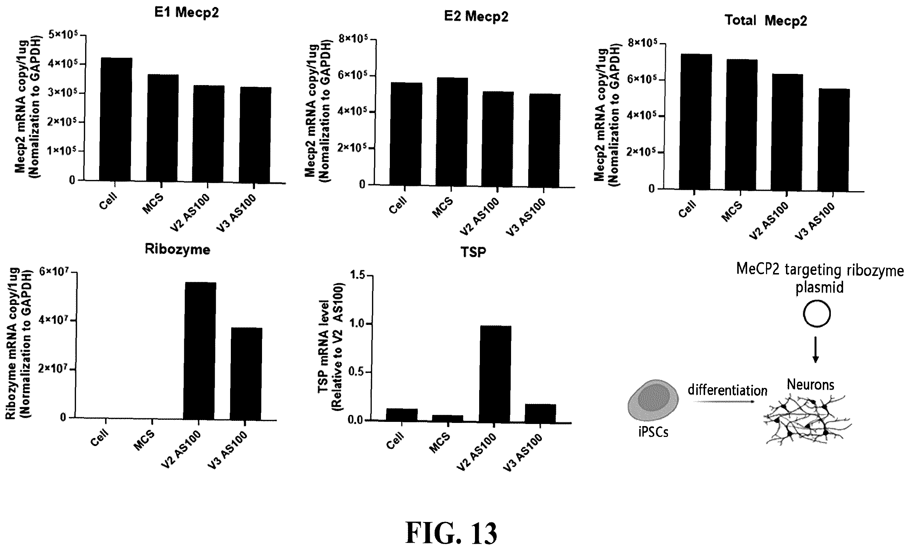

As a result, although the reduction effect on MeCP2 was not large, it was confirmed that the expression of neuronal MeCP2 was reduced and that a trans-splicing product was generated (FIG. 13).

Therefore, the effect of the prepared MeCP2-targeting ribozyme was also confirmed on MeCP2 expressed in neurons.

Although the embodiments have been described with reference to the limited drawings, one of ordinary skill in the art may apply various technical modifications and variations based thereon. For example, appropriate results may be achieved if the described techniques are performed in a different order, and/or if components in a described system, structure, device, or circuit are combined in a different manner, and/or replaced or substituted by other components or their equivalents.

Therefore, other implementations, other embodiments, and equivalents to the claims are also within the scope of the following claims.

## Claims

1. A trans-splicing ribozyme targeting an MeCP2 transcript.

2. The trans-splicing ribozyme of claim 1, wherein the trans-splicing ribozyme has a structure of 5'-IGS (internal guide sequence)-Ribozyme*-exon-3'.

3. The trans-splicing ribozyme of claim 2, wherein the IGS region comprises a nucleotide sequence of 2 to 10 nt in length capable of binding complementarily to a region comprising base +18, +31, +33, +36, +45, +54, +127, +145, +148, +162, +167, +181, +188, +211, +294, +289, +296, +304, +314, or +357 of the MeCP2 transcript represented by SEQ ID NO: 1.

4. The trans-splicing ribozyme of claim 2, wherein the IGS region comprises the nucleotide sequence of SEQ ID NO: 3, 5, 7, 9, 11, 13, 15, 17, 19, 21, 23, 25, 27, 29, 31, 33, 35, 37, 39, or 61.

5. The trans-splicing ribozyme of claim 2, wherein the trans-splicing ribozyme further comprises an antisense sequence (AS) region capable of binding complementarily to a portion of the MeCP2 transcript in a 5'-terminal direction of the trans-splicing ribozyme.

6. The trans-splicing ribozyme of claim 2, wherein the exon region comprises a polynucleotide sequence encoding a normal MeCP2 protein or a nucleotide sequence encoding a reporter protein.

7. The trans-splicing ribozyme of claim 2, wherein the trans-splicing ribozyme further comprises a nucleotide sequence extended in a 5' direction of the IGS region,
a portion of the extended nucleotide sequence consists of a nucleotide sequence capable of binding complementarily to an MeCP2 RNA together with the IGS region to form a P1 helix, and
a portion of the extended nucleotide sequence consists of a nucleotide sequence capable of binding complementarily to a nucleotide sequence in a 5' direction of the exon region to form a P10 helix.

8. The trans-splicing ribozyme of claim 1, wherein the MeCP2 transcript comprises an MeCP2 mutation.

9. The trans-splicing ribozyme of claim 8, wherein the MeCP2 mutation has a substitution, deletion, or addition occurring at one or more bases among bases 1 to 10467 of the MeCP2 transcript represented by SEQ ID NO: 1.

10. A trans-splicing ribozyme expression cassette encoding the trans-splicing ribozyme of any one of claims 1 to 9.

11. The trans-splicing ribozyme expression cassette of claim 10, comprising a promoter sequence operatively linked to the trans-splicing ribozyme expression cassette.

12. A trans-splicing ribozyme expression vector comprising the trans-splicing ribozyme expression cassette of claim 10 or 11.

13. A recombinant virus comprising the trans-splicing ribozyme expression cassette of claim 10 or 11.

14. The recombinant virus of claim 13, wherein the virus is any one selected from the group consisting of an adenovirus, an adeno-associated virus (AAV), a retrovirus, a lentivirus, a herpes simplex virus, and a vaccinia virus.

15. A non-viral gene delivery vehicle comprising the trans-splicing ribozyme expression cassette of claim 10 or 11.

16. A pharmaceutical composition for preventing or treating Rett syndrome, the pharmaceutical composition comprising, as an active ingredient, any one selected from the group consisting of the trans-splicing ribozyme of claims 1 to 9, the trans-splicing ribozyme expression cassette of claims 10 and 11, the expression vector of claim 12, and a gene delivery system comprising the expression vector.
